# FASCICULE DE BREVET EUROPEEN

(11) **EP 3 316 931 B1**
(45) Date de publication et mention de la délivrance du brevet: **27.03.2019**
(21) Numéro de dépôt: 16742360.7
(22) Date de dépôt: 30.06.2016
(51) Int. Cl.: A61M 5/20, A61M 5/30, A61M 5/315

(54) **DISPOSITIF D'INJECTION SANS AIGUILLE ÉQUIPÉ D'UN DISPOSITIF DE PERCUSSION PERFECTIONNÉ**
NADELLOSER INJEKTOR MIT VERBESSERTER SCHLAGVORRICHTUNG
NEEDLELESS INJECTOR WITH IMPROVED PERCUSSIVE DEVICE

(30) Priorité: 30.06.2015 FR 1556159
(43) Date de publication de la demande: 09.05.2018
(73) Titulaire: Crossject, 21000 Dijon (FR)
(72) Inventeur: VIVIEN, Gilles, 92240 Malakof (FR); VIGOT, Xavier, 21260 Veronnes (FR)
(74) Mandataire: Chevalier, Renaud Philippe
(86) Numéro de dépôt international: PCT/FR2016/051655
(87) Numéro de publication internationale: WO 2017/001794

(56) Documents cités:
- DE-A1-102007 031 714
- FR-A1- 2 815 544
- US-A1- 2013 237 921

## Description

L'invention concerne un dispositif d'injection sans aiguille équipé d'un dispositif de percussion d'un générateur de gaz.

Le domaine technique de l'invention est celui des dispositifs d'injection sans aiguille, pré-remplis et jetables, fonctionnant avec une source d'énergie comme par exemple un générateur de gaz, et utilisés pour les injections intradermiques, sous-cutanées et intramusculaires, de principe actif liquide à usage thérapeutique en médecine humaine ou vétérinaire.

Le principe actif est constitué par un liquide plus ou moins visqueux, un mélange de liquide, ou un gel. Le principe actif peut également être un solide mis en solution dans un solvant approprié pour l'injection ou être constitué d'un solide pulvérulent mis en suspension à une certaine concentration dans un liquide approprié. La granulométrie du principe actif doit alors être compatible avec le diamètre des conduits pour éviter de les obturer.

Un dispositif d'injection comporte de manière connue, comme par exemple dans la demande de brevet FR-A-2815544 (équivalente à WO 02/34317), un corps comprenant successivement un générateur de gaz, une chambre d'expansion, un réservoir contenant le principe actif liquide et un système d'injection.

Le réservoir est constitué par un tube en verre qui est inséré dans le corps du dispositif et qui est obturé par un bouchon-piston amont et un bouchon-piston aval entre lesquels est contenu le principe actif liquide.

L'extrémité libre aval du réservoir coopère avec une buse d'injection qui délimite au moins un canal d'injection s'étendant axialement suivant un axe d'injection.

De plus, le dispositif d'injection comporte un capot creux qui enveloppe le corps et qui délimite une ouverture inférieure adaptée pour le passage de la buse d'injection.

L'extrémité libre de la buse d'injection qui fait saillie hors du corps et du capot est protégée par un opercule amovible et un bouchon amovible qui loge l'opercule.

Pour permettre l'injection du principe actif, le corps est monté coulissant dans le capot, de bas en haut suivant un axe de coulissement, entre une position de repos et une position d'injection, l'entraînement du corps étant réalisé lorsque le patient appui la buse d'injection sur sa peau.

Le déplacement du corps dans le capot permet le déclenchement du générateur de gaz générant un gaz sous pression qui entraîne en déplacement les bouchon-pistons pour injecter le principe actif à travers la peau du patient en passant par la buse d'injection.

A cet effet, le dispositif d'injection est équipé d'un dispositif de percussion qui comporte un percuteur monté coulissant suivant un axe de coulissement, entre une position de repos dans laquelle le percuteur est bloqué en translation par un moyen de verrouillage à l'encontre d'un ressort précontraint, et une position de percussion dans laquelle le percuteur est libéré par le moyen de verrouillage pour percuter une amorce du générateur de gaz sous l'action du ressort.

Le document FR-A-2815544 décrit et représente un moyen de verrouillage qui comporte deux protubérances qui s'étendent radialement depuis la face périphérique du percuteur et qui présentent chacune une rampe du type hélicoïdale.

De façon complémentaire, le capot présente deux ergots qui présentent chacun une rampe du type hélicoïdale, les rampes du capot étant adaptées pour être en appui sur les rampes associées du percuteur.

Les rampes sont conçues pour permettre, par frottement, l'entraînement du percuteur suivant un mouvement combiné de translation et de pivotement autour de l'axe de coulissement, lorsque le corps est entraîné vers sa position d'injection.

Le pivotement du percuteur permet au percuteur de se libérer d'une entrave qui s'oppose à son coulissement axial, à l'encontre du ressort précontraint.

Une fois libéré de son entrave, le percuteur est entraîné avec force vers sa position de percussion, par le ressort précontraint prévu à cet effet, pour percuter l'amorce du générateur de gaz et déclencher le générateur de gaz.

Un inconvénient de ce type de dispositif de percussion est le frottement important du percuteur sur les rampes associées du capot qui entraîne une perte d'énergie et qui peut parfois entraîner un blocage du dispositif.

De plus, les efforts radiaux exercés sur les pattes de retenue par le percuteur peuvent provoquer des déformations plastiques si l'effort de poussée induit par le ressort est trop important.

La présente invention vise notamment à résoudre ces inconvénients en proposant un dispositif de percussion plus fiable et limitant les frottements.

Dans ce but, l'invention se rapporte à un dispositif d'injection sans aiguille comportant :
- un capot,
- un système d'injection qui comprend au moins un piston, un réservoir de principe actif, une buse d'injection délimitant au moins un canal d'injection,
- un corps qui est enveloppé par le capot et qui est monté coulissant par rapport au capot, de bas en haut suivant un axe de coulissement, entre une position de repos et une position d'injection,
- un générateur de gaz comprenant une amorce, et
- un dispositif de percussion qui comporte un percuteur monté coulissant suivant l'axe de coulissement, entre une position de repos dans laquelle le percuteur est bloqué en translation par un moyen de verrouillage à l'encontre d'un ressort précontraint, et une position de percussion dans laquelle le percuteur est libéré par le moyen de verrouillage pour percuter l'amorce du générateur de gaz sous l'action dudit ressort,
caractérisé en ce que le moyen de verrouillage du percuteur comporte au moins :
- un ergot qui fait saillie depuis une face périphérique du percuteur suivant un axe globalement perpendiculaire à l'axe de coulissement et qui présente une première rampe d'appui,
- une patte de retenue qui est solidaire du capot et qui présente une seconde rampe d'appui agencée en regard de ladite première rampe d'appui, lesdites rampes étant conçues pour coopérer ensemble afin d'entraîner la patte de retenue radialement sous l'effet de la poussée du percuteur, depuis une position de retenue dans laquelle la première rampe est en appui axial sur la seconde rampe de sorte que la patte de retenue s'oppose au coulissement du percuteur, jusqu'à une position de relâchement dans laquelle la patte (82a) de retenue est écartée pour libérer le percuteur, et
- une entrave qui est solidaire du corps et qui est conçue pour contraindre la patte de retenue dans sa position de retenue, lorsque le corps occupe sa position de repos, et pour libérer la patte de retenue dans sa position de relâchement lorsque le corps occupe sa position d'injection.

Avantageusement, l'invention permet de limiter le mouvement du percuteur à un déplacement linéaire pour réduire les frottements entre le percuteur et la patte de retenue.

Plus particulièrement, la première rampe et la seconde rampe s'étendent chacune globalement radialement en présentant une inclinaison vers le bas qui est adaptée pour permettre à la seconde rampe de la patte de retenue de glisser sur la première rampe de l'ergot et d'écarter la patte de retenue vers sa position de relâchement, sous l'effet de la poussée axiale du percuteur entraîné par le ressort associé.

A l'inverse, l'ergot du percuteur présente une première contre-rampe et la patte de retenue présente une seconde contre-rampe, lesdites contre-rampes s'étendent chacune globalement radialement en présentant une inclinaison vers le haut qui est adaptée pour permettre à la seconde contre-rampe de la patte de retenue de glisser sur la première contre-rampe de l'ergot et d'entraîner la patte de retenue vers sa position de relâchement, lorsque le percuteur est entraîné en coulissement depuis sa position de percussion, jusqu'à sa position de percussion.

Selon un autre aspect, le capot comprend une semelle, formant fond de capot, qui s'étend perpendiculairement à l'axe de coulissement, et la patte de retenue s'étend axialement suivant l'axe de coulissement depuis une base reliée sur ladite semelle, jusqu'à une tête qui délimite la seconde rampe, la patte de retenue étant déformable élastiquement radialement pour permettre l'écartement de la patte depuis sa position de retenue jusqu'à sa position de relâchement.

De plus, le ressort précontraint qui coopère avec le percuteur est interposé axialement, suivant l'axe de coulissement, entre la semelle du capot et une face d'appui du percuteur.

Avantageusement, la patte de retenue est réalisée en matériau plastique venue de matière avec la semelle du capot.

Aussi, le corps délimite un fourreau de guidage du percuteur, qui présente une forme globalement tubulaire suivant l'axe de coulissement et qui est conçu pour guider axialement le percuteur entre sa position de repos et sa position de percussion.

De plus, le fourreau de guidage délimite au moins une rainure axiale qui forme l'entrave de la patte de retenue et qui est adaptée pour former un appui radial d'une extrémité libre axiale de la patte de retenue.

En outre, une extrémité libre axiale du percuteur forme un téton adapté pour percuter et initier l'amorce du générateur de gaz.

De préférence, le dispositif de percussion présente une conception symétrique suivant un plan axial passant par l'axe de coulissement, ledit dispositif comportant, par symétrie, deux pattes de retenue formant pince qui coopèrent chacune avec un ergot associé du percuteur.

Selon un exemple de réalisation, le principe actif contenu dans le réservoir est choisi parmi le groupe comprenant les principes actifs suivants :
- Methotrexate,
- Adrénaline,
- Sumatriptan,
- Hydrocortisone,
- Naloxone,
- Midazolam,
- Apomorphine,
- Ethylnatrexone bromide,
- Phytomenadione,
- Chlorpromazine hydrocloride,
- Zuclopenthixol acetate,
- Danaparoid sodium,
- Enoxaparin sodium,
- Estradiol cypionate,
- Medoxyprogesterone acetate,
- Medroparin calcium,
- Methylprednisolone acetate
- Heparin calcium,
- Terbuline.

D'autres caractéristiques et avantages de l'invention apparaîtront à la lecture de la description détaillée qui suit pour la compréhension de laquelle on se reportera aux dessins annexés dans lesquels :
- la figure 1 est une vue en perspective éclatée axialement, qui illustre un dispositif d'injection sans aiguille comportant un dispositif de percussion, selon l'invention ;
- la figure 2 est une vue en coupe axiale transversale qui illustre le percuteur du dispositif de percussion de la figure 1 monté coulissant dans le corps ;
- la figure 3 est une vue en perspective de détail en coupe axiale, qui illustre le percuteur et les pattes de retenues du dispositif de la figure 1 ;
- les figures 4, 5 et 6 sont des vues schématiques en section axiale transversale, qui illustrent les étapes successives du déclenchement du dispositif de percussion.

Dans la description et les revendications, pour clarifier la description et les revendications, on adoptera à titre non limitatif la terminologie longitudinal, vertical et transversal en référence au trièdre L, V, T indiqué aux figures.

De plus, dans la présente demande, les termes « haut », « bas », « supérieur », « inférieur », « horizontal », « vertical », et leurs dérivés font référence à la position ou à l'orientation d'un élément ou d'un composant, cette position ou cette orientation étant considérée en référence à l'orientation du dispositif sur les figures et au trièdre L, V, T, sans référence à la gravité terrestre.

De même, les termes « axial » et « radial » doivent s'entendre en référence à l'axe A de coulissement vertical du corps du dispositif d'injection.

Aussi, pour faciliter la compréhension de la description, les éléments identiques et symétriques suivant le plan P de symétrie sont indiqués par des mêmes références numériques distinguées par la lettre « a » ou « b ».

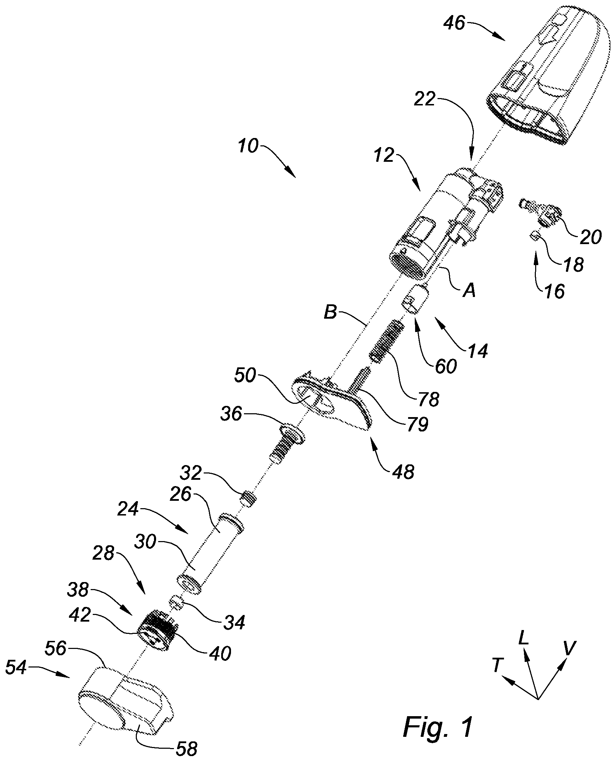

On a représenté à la figure 1 un dispositif 10 d'injection sans aiguille, ou seringue sans aiguille, qui comporte un corps 12 en forme de U comprenant successivement un dispositif de percussion 14, un générateur de gaz 16 comprenant une amorce 18 et une charge pyrotechnique 20, une chambre d'expansion 22, un réservoir 24 contenant le principe actif liquide 26 et un système d'injection 28.

Le dispositif de percussion 14 et le générateur de gaz 16 constituent un premier sous-ensemble linéaire du corps 12 qui s'étend axialement suivant un axe A de coulissement vertical, et le réservoir 24 contenant le principe actif 26 et le système d'injection 28 forment un deuxième sous-ensemble linéaire du corps 12 qui s'étend axialement suivant un second axe B d'injection vertical.

Selon un exemple de réalisation préféré, le corps 12 est réalisé en matériau plastique.

Ces deux sous-ensembles sont reliés l'un à l'autre par la chambre d'expansion 22 qui a un axe perpendiculaire aux axes A, B des sous-ensembles.

Le réservoir 24 est constitué par un tube 30 en verre obturé par un bouchon-piston amont 32 et un bouchon-piston aval 34 entre lesquels est contenu le principe actif liquide 26, les bouchons-pistons étant réalisés en matériau déformable élastiquement à base d'élastomère.

Le réservoir 24 est inséré dans le corps 12 et est bloqué verticalement, d'une part, à sa partie amont par une pièce cylindrique 36 munie d'une ouverture centrale permettant de mettre en communication le bouchon-piston amont 32 avec la chambre d'expansion 22, et, d'autre part, à sa partie aval par une buse 38 d'injection.

La buse 38 présente une forme cylindrique suivant l'axe B d'injection qui est délmitée par une face cylindrique périphérique 40 munie d'un filetage, le filetage étant prévu pour coopérer avec un taraudage complémentaire formé sur la paroi interne de l'extrémité aval du corps 12.

De plus, la buse 38 délimite trois canaux 42 axiaux d'injection parallèles à l'axe B d'injection, illustrés à la figure 1.

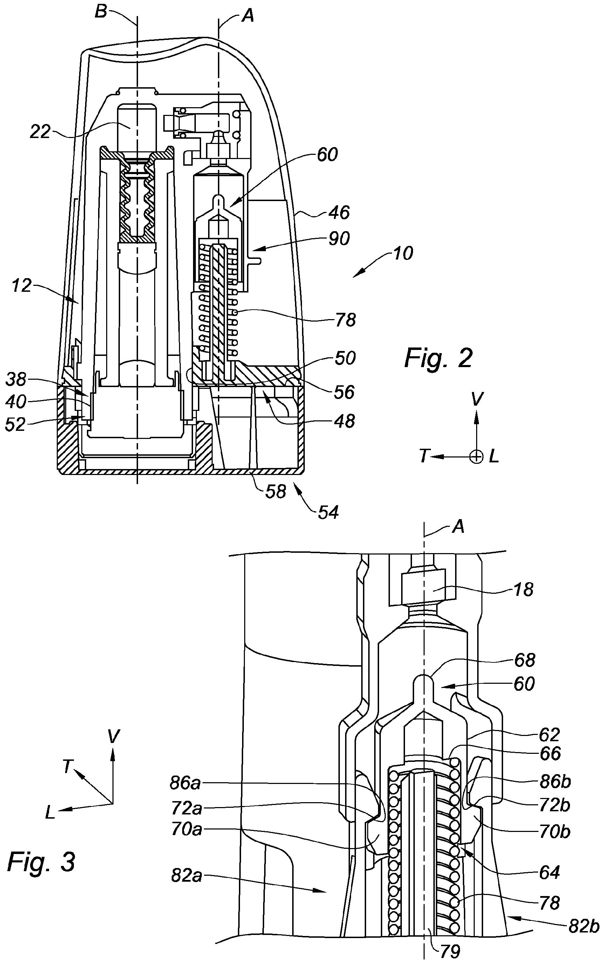

En référence aux figures 1 et 2, le corps 12 est enveloppé par un capot 46 creux qui délimite une ouverture inférieure fermée par une semelle 48 horizontale formant fond de capot.

La semelle 48 délimite un passage circulaire 50 autour de l'axe B d'injection qui est adapté pour le passage de la buse 38 d'injection et de l'extrémité aval du corps 12, de sorte que la buse 38 comporte un tronçon inférieur 52 faisant saillie verticalement vers le bas hors du capot 46.

Aussi, le dispositif 10 d'injection est équipé d'un bouchon 54 qui est délimité verticalement par une face supérieure 56 ouverte en appui sur la semelle 48 du capot 46, et une face inférieure 58 fermée globalement plane.

Le bouchon 54 est monté sur le corps 12 de façon amovible par un moyen de verrouillage du type à baïonnette.

Selon un autre aspect, le dispositif de percussion 14 comporte un percuteur 60 qui présente une forme globalement cylindrique borgne suivant l'axe A de coulissement et qui est délimité par une paroi périphérique 62 cylindrique.

Selon un exemple de réalisation préféré, le percuteur 60 est réalisé en matériau métallique, par exemple en alliage connu sous l'acronyme « ZAMAK ».

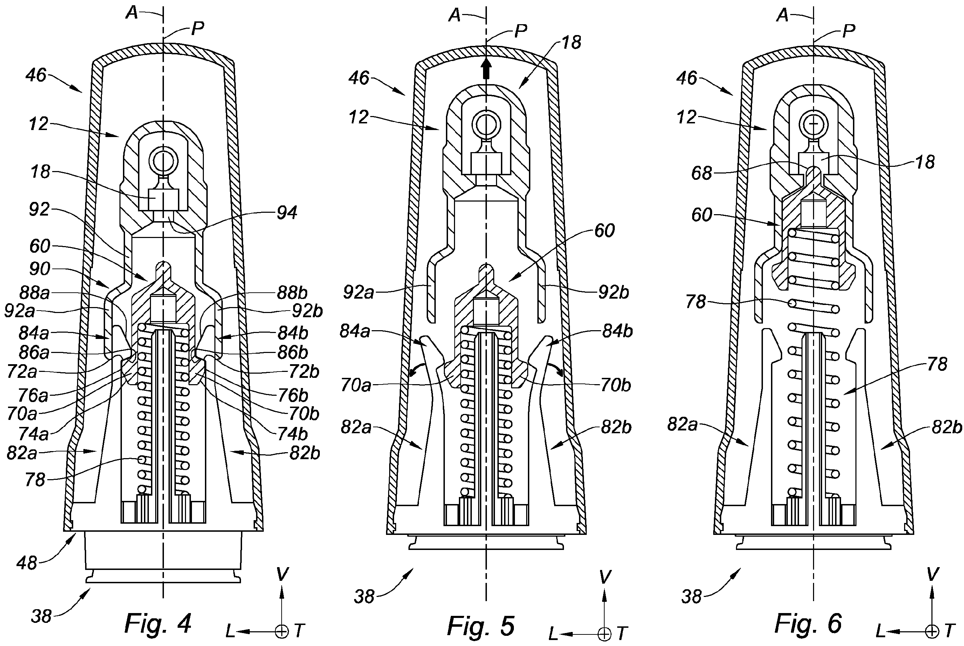

Le dispositif de percussion 14 présente une conception symétrique suivant un plan P axial longitudinal de symétrie, passant par l'axe A de coulissement, illustré aux figures 4 à 6.

Comme on peut le voir à la figure 3, le percuteur 60 forme un logement 64 interne borgne qui débouche axialement à l'extrémité libre inférieure du percuteur 60 et qui est délimité par un siège 66 annulaire supérieur.

De plus, le percuteur 60 présente, à son extrémité axiale libre supérieure, un téton 68 adapté pour percuter et initier l'amorce 18 du générateur de gaz 16.

Aussi, le percuteur 60 est équipé de deux ergots 70a, 70b opposés symétriques qui font saillie radialement depuis la face périphérique 62 du percuteur 60 et qui présentent chacun une première rampe 72a, 72b supérieure d'appui respectivement.

Les premières rampes 72a, 72b s'étendent chacune globalement radialement depuis la face périphérique 62 du percuteur 60, en présentant une inclinaison vers le bas. L'angle d'inclinaison est, selon un exemple de réalisation, environ de vingt degrés par rapport à un plan radial horizontal non incliné.

De même, les ergots 70a, 70b du percuteur 60 présentent chacun une première contre-rampe 74a, 74b inférieure respectivement, qui s'étendent chacune globalement radialement depuis la face périphérique 62 du percuteur 60, en présentant une inclinaison vers le haut. L'angle d'inclinaison est, selon un exemple de réalisation, environ de soixante degrés par rapport à un axe radial horizontal non incliné.

Les premières rampes 72a, 72b et les premières contre-rampes 74a, 74b sont reliées entre elles par une portion axiale 76a, 76b respectivement.

Selon la figure 4, le percuteur 60 coopère avec un ressort 78 hélicoïdal précontraint qui est interposé axialement, suivant l'axe A de coulissement, entre une face supérieure de la semelle 48 du capot 46 et le siège annulaire 66 du percuteur 60.

Le ressort 78 est enfilé sur un mat 79 qui s'étend axialement depuis la semelle 48 et qui est conçu pour guider le ressort 78 suivant l'axe A de coulissement.

A titre non limitatif, le ressort 78 peut être remplacé par tout autre moyen de rappel élastique, comme un ressort à gaz par exemple.

Complémentairement, le dispositif de percussion 14 comporte deux pattes 82a, 82b de retenue, illustrées à la figure 4, qui sont agencées de part et d'autre du plan P de symétrie.

Chaque patte 82a, 82b de retenue s'étend axialement, suivant l'axe A de coulissement, depuis une base reliée sur la semelle 48 du capot 46, jusqu'à une tête 84a, 84b respectivement qui forme un cran sailliant radialement vers l'axe A de coulissement.

Chaque tête 84a, 84b délimite une seconde rampe 86a, 86b respectivement, les secondes rampes 86a, 86b s'étendant globalement en regard et parallèlement aux premières rampes 72a, 72b respectivement du percuteur 60.

De même, chaque tête 84a, 84b délimite une seconde contre-rampe 88a, 88b respectivement, les secondes contre-rampes 88a, 88b s'étendant globalement parallèlement aux premières contre-rampes 74a, 74b respectivement du percuteur 60.

Selon un exemple de réalisation préféré, chaque patte 82a, 82b de retenue est réalisée en matériau plastique venue de matière avec la semelle 48 du capot 46.

Comme on peut le voir à la figure 4, le corps 12 délimite un fourreau 90 de guidage du percuteur 60, qui présente une forme globalement tubulaire suivant l'axe A de coulissement et qui est conçu pour guider axialement le percuteur 60.

A cet effet, le fourreau 90 présente un tronçon de guidage 92 cylindrique qui est ouvert axialement vers le bas pour permettre le passage du percuteur 60 et qui délimite un passage 94 supérieur d'accès à l'amorce 18 de la charge pyrotechnique 20.

De plus, le fourreau 90 délimite deux rainures axiales symétriques formant entrave 92a, 92b respectivement, qui sont conçues pour former un appui radial des têtes 84a, 84b des pattes 82a, 82b de retenue respectivement.

L'ensemble constitué par les ergots 70a, 70b du percuteur 60, les pattes 82a, 82b de retenue et les entraves 92a, 92b forme un moyen de verrouillage du percuteur 60.

Pour permettre le déclenchement du dispositif de percussion 14, plusieurs éléments sont mobiles, comme on peut le comprendre à lecture de la description suivante, en référence aux figures 4 à 6.

Le corps 12 est monté coulissant par rapport au capot 46, de bas en haut suivant l'axe A de coulissement, entre une position de repos, illustrée à la figure 4, dans laquelle le dispositif de percussion 14 n'est pas déclenché, et une position d'injection, représentée à la figure 6, dans laquelle le corps 12 est coulissé axialement vers le haut et le dispositif de percussion 14 est déclenché.

La buse 38 d'injection étant solidaire du corps 12, le corps 12 est entraîné vers sa position d'injection lorsque le patient applique la buse 38 sur sa peau et entraîne le capot 46 vers le bas, en direction de sa peau.

Aussi, le percuteur 60 est monté coulissant suivant l'axe A de coulissement, entre une position de repos illustrée à la figure 4, dans laquelle le percuteur 60 est bloqué en translation par le moyen de verrouillage à l'encontre du ressort 78 précontraint, et une position de percussion illustrée à la figure 6, dans laquelle le percuteur 60 est libéré par le moyen de verrouillage pour percuter l'amorce 18 du générateur de gaz 16 sous l'action du ressort 78.

En effet, comme on peut le voir à la figure 4, les secondes rampes 86a, 86b d'appui formées par les pattes 82a, 82b de retenue sont en appui axial sur les première rampes 72a, 72b d'appui formées par les ergots 70a, 70b du percuteur 60 pour s'opposer au coulissement axial du percuteur 60 vers le haut, à l'encontre du ressort 78.

Complémentairement, la tête 84a, 84b de chaque patte 82a, 82b de retenue est bloquée radialement par les entraves 92a, 92b prévues à cet effet, pour empêcher l'écartement des pattes 82a, 82b.

La figure 5 illustre une étape intermédiaire de coulissement du corps 12, entre sa position de repos et sa position d'injection, étape au cours de laquelle les entraves 92a, 92b coulissent vers le haut et libèrent les pattes 82a, 82b qui s'écartent radialement vers l'extérieur sous l'effet de l'appui axial des premières rampes 72a, 72b du percuteur 60 sur les secondes rampes 86a, 86b des pattes 82a, 82b.

En effet, l'inclinaison des rampes 72a, 72b, 86a, 86b est adaptée pour faire glisser et écarter les pattes 82a, 82b de retenue radialement vers l'extérieur.

Dans ce but, on comprend que les pattes 82a, 82b de retenue sont déformables élastiquement radialement entre une position de retenue illustrée à la figure 4, et une position de relâchement illustrée à la figure 5.

La figure 6 illustre la fin de course du percuteur 60 qui occupe sa position de percussion dans laquelle le téton 68 percute et initie l'amorce 18 pour déclencher le générateur de gaz 16 et l'injection du principe actif.

Ainsi, l'invention permet de limiter les efforts de frottement qui s'opposent au coulissement du percuteur 60, notamment en réduisant le mouvement du percuteur 60 à un mouvement linéaire.

De plus, les surfaces de frottement délimitées par les premières rampes 72a, 72b et les secondes rampes 86a, 86b sont relativement faibles.

Il est possible de réduire ces surfaces de frottement en jouant sur les formes des rampes pour limiter l'appui des rampes les unes sur les autres à un appui linéaire, par exemple en donnant une forme bombée convexe aux première rampes 72a, 72b et une forme plane aux secondes rampes 86a, 86b associées.

De même, l'invention évite d'éventuelles déformations plastiques des pattes 82a, 82b de retenue lorsque le percuteur 60 occupe sa position de repos, position dans laquelle le percuteur exerce un effort d'écartement radial sur la tête des pattes 82a, 82b de retenue.

En effet, les entraves 92a, 92b sont en appui radial sur toute la longueur des têtes 84a, 84b respectivement, empêchant ainsi la torsion des têtes 84a, 84b lorsque le percuteur 60 occupe sa position de repos.

Ces caractéristiques permettent de réaliser le corps 12 en matériau plastique, ce qui présente un gain de masse par rapport à un corps réalisé en métal.

Selon un autre aspect, le dispositif de déclenchement est adapté pour favoriser le montage du percuteur 60 dans sa position de repos, par la présence des premières contre-rampes 74a, 74b du percuteur 60 et des secondes contre-rampes 88a, 88b des pattes 82a, 82b de retenue, qui permettent l'écartement des pattes 82a, 82b au cours du coulissement du percuteur 60 vers le bas depuis sa position de percussion jusqu'à sa position de repos.

On notera également que lorsque le dispositif de percussion a été déclenché, il n'est pas possible de repositionner le percuteur dans sa position d'origine dans laquelle il est retenu par les pattes 82a, 82b de retenue.

Cette caractéristique assure que le dispositif 10 d'injection ne peut pas être réutilisé.

## Revendications

1. Dispositif (10) d'injection sans aiguille comportant :
- un capot (46),
- un système d'injection (28) qui comprend au moins un piston (32, 34), un réservoir (24) contenant un principe actif, une buse (38) d'injection délimitant au moins un canal (42) d'injection,
- un corps (12) qui est enveloppé par le capot (46) et qui est monté coulissant par rapport au capot (46), de bas en haut suivant un axe (A) de coulissement, entre une position de repos et une position d'injection,
- un générateur de gaz (16) comprenant une amorce (18), et
- un dispositif (14) de percussion qui comporte un percuteur (60) monté coulissant suivant l'axe (A) de coulissement, entre une position de repos dans laquelle le percuteur (60) est bloqué en translation par un moyen de verrouillage à l'encontre d'un ressort (78) précontraint, et une position de percussion dans laquelle le percuteur (60) est libéré par le moyen de verrouillage pour percuter l'amorce (18) du générateur de gaz (16) sous l'action dudit ressort (78),
**caractérisé en ce que** le moyen de verrouillage du percuteur (60) comporte au moins :
- un ergot (70a) qui fait saillie depuis une face périphérique (62) du percuteur (60) suivant un axe globalement perpendiculaire à l'axe (A) de coulissement et qui présente une première rampe (72a) d'appui,
- une patte (82a) de retenue qui est solidaire du capot (46) et qui présente une seconde rampe (86a) d'appui agencée en regard de ladite première rampe (72a) d'appui, lesdites rampes (72a, 86a) étant conçues pour coopérer ensemble afin d'entraîner la patte (82a) de retenue radialement sous l'effet de la poussée du percuteur (60), depuis une position de retenue dans laquelle la première rampe (72a) est en appui axial sur la seconde rampe (86a) de sorte que la patte (82a) de retenue s'oppose au coulissement du percuteur (60), jusqu'à une position de relâchement dans laquelle la patte (82a) de retenue est écartée pour libérer le percuteur (60), et
- une entrave (92a) qui est solidaire du corps (12) et qui est conçue pour contraindre la patte (82a) de retenue dans sa position de retenue, lorsque le corps (12) occupe sa position de repos, et pour libérer la patte (82a) de retenue dans sa position de relâchement lorsque le corps (12) occupe sa position d'injection.

2. Dispositif (10) d'injection sans aiguille selon la revendication 1, **caractérisé en ce que** la première rampe (72a) et la seconde rampe (86a) s'étendent chacune globalement radialement en présentant une inclinaison vers le bas qui est adaptée pour permettre à la seconde rampe (86a) de la patte (82a) de retenue de glisser sur la première rampe (72a) de l'ergot (60) et d'écarter la patte (82a) de retenue vers sa position de relâchement, sous l'effet de la poussée axiale du percuteur (60) entraîné par le ressort (78) associé.

3. Dispositif (10) d'injection sans aiguille selon l'une quelconque des revendications précédentes, **caractérisé en ce que** l'ergot (70a) du percuteur (60) présente une première contre-rampe (74a) et la patte (82a) de retenue présente une seconde contre-rampe 88a, lesdites contre-rampes s'étendent chacune globalement radialement en présentant une inclinaison vers le haut qui est adaptée pour permettre à la seconde contre-rampe (88a) de la patte (82a) de retenue de glisser sur la première contre-rampe (74a) de l'ergot (70a) et d'entraîner la patte (82a) de retenue vers sa position de relâchement, lorsque le percuteur (60) est entraîné en coulissement depuis sa position de percussion, jusqu'à sa position de percussion.

4. Dispositif (10) d'injection sans aiguille selon l'une quelconque des revendications précédentes, **caractérisé en ce que** le (46) capot comprend une semelle (48), formant fond de capot, qui s'étend perpendiculairement à l'axe (A) de coulissement, et **en ce que** la patte (82a) de retenue s'étend axialement suivant l'axe (A) de coulissement depuis une base reliée sur ladite semelle (48), jusqu'à une tête (84a) qui délimite la seconde rampe (86a), la patte (82a) de retenue étant déformable élastiquement radialement pour permettre l'écartement de la patte (82a) depuis sa position de retenue jusqu'à sa position de relâchement.

5. Dispositif (10) d'injection sans aiguille selon la revendication 4, **caractérisé en ce que** le ressort (78) précontraint qui coopère avec le percuteur (60) est interposé axialement, suivant l'axe (A) de coulissement, entre la semelle (48) du capot (46) et une face d'appui (66) du percuteur (60).

6. Dispositif (10) d'injection sans aiguille selon la revendication 4, **caractérisé en ce que** la patte (82a) de retenue est réalisée en matériau plastique venue de matière avec la semelle (48) du capot (46).

7. Dispositif (10) d'injection sans aiguille selon l'une quelconque des revendications précédentes, **caractérisé en ce que** le corps (12) délimite un fourreau (90) de guidage du percuteur (60), qui présente une forme globalement tubulaire suivant l'axe (A) de coulissement et qui est conçu pour guider axialement le percuteur (60) entre sa position de repos et sa position de percussion.

8. Dispositif (10) d'injection sans aiguille selon la revendication 7, **caractérisé en ce que** le fourreau (90) de guidage délimite au moins une rainure (92a) axiale qui forme l'entrave (92a) de la patte (82a) de retenue et qui est adaptée pour former un appui radial d'une extrémité libre axiale de la patte (82a) de retenue.

9. Dispositif (10) d'injection sans aiguille selon l'une quelconque des revendications précédentes, **caractérisé en ce que** une extrémité libre axiale du percuteur (60) forme un téton (68) adapté pour percuter et initier l'amorce (18) du générateur de gaz (16).

10. Dispositif (10) d'injection sans aiguille selon l'une quelconque des revendications précédentes, **caractérisé en ce que** le dispositif de percussion (14) présente une conception symétrique suivant un plan (P) axial passant par l'axe (A) de coulissement, ledit dispositif (14) comportant, par symétrie, deux pattes (82a, 82b) de retenue formant pince qui coopèrent chacune avec un ergot (70a, 70b) associé du percuteur (60).

11. Dispositif (10) d'injection sans aiguille selon l'une quelconque des revendications précédentes, **caractérisé en ce que** le principe actif (26) contenu dans le réservoir (24) est choisi parmi le groupe comprenant les principes actifs suivants :
- Methotrexate,
- Adrénaline,
- Sumatriptan,
- Hydrocortisone,
- Naloxone,
- Midazolam,
- Apomorphine,
- Ethylnatrexone bromide,
- Phytomenadione,
- Chlorpromazine hydrocloride,
- Zuclopenthixol acetate,
- Danaparoid sodium,
- Enoxaparin sodium,
- Estradiol cypionate,
- Medoxyprogesterone acetate,
- Medroparin calcium,
- Methylprednisolone acetate
- Heparin calcium,
- Terbuline.

## Patentansprüche

1. Vorrichtung (10) zum Einspritzen ohne Nadel, die Folgendes umfasst:
- eine Haube (46),
- ein Einspritzsystem (28), das mindestens einen Kolben (32, 34), einen Behälter (24), der einen Wirkstoff enthält, eine Einspritzdüse (38), die mindestens einen Einspritzkanal (42) abgrenzt, umfasst,
- einen Körper (12), der von der Haube (46) umgeben ist und der bezüglich der Haube (46) von unten nach oben entlang einer Gleitachse (A) zwischen einer Ruheposition und einer Einspritzposition gleitend montiert ist,
- einen Gasgenerator (16), der ein Zündelement (18) umfasst, und
- eine Schlagvorrichtung (14), die einen Schlagbolzen (60) umfasst, der entlang der Gleitachse (A) zwischen einer Ruheposition, in der der Schlagbolzen (60) in Verschiebung durch ein Verriegelungsmittel gegen eine vorgespannte Feder (78) blockiert ist, und einer Schlagposition, in der der Schlagbolzen (60) durch das Verriegelungsmittel freigegeben wird, um das Zündelement (18) des Gasgenerators (16) unter der Einwirkung der Feder (78) zu schlagen, gleitend montiert ist,
**dadurch gekennzeichnet, dass** das Verriegelungsmittel des Schlagbolzens (60) mindestens Folgendes umfasst:
- einen Dorn (70a), der von einer Umfangsfläche (62) des Schlagbolzens (60) entlang einer im Allgemeinen zu der Gleitachse (A) senkrechten Achse vorragt und der eine erste Auflagerampe (72a) aufweist,
- eine Rückhaltepratze (82a), die mit der Haube (46) fest verbunden ist und die eine zweite Auflagerampe (86a) aufweist, die gegenüber der ersten Auflagerampe (72a) eingerichtet ist, wobei die Rampen (72a, 86a) ausgelegt sind, um miteinander zusammenzuwirken, um die Rückhaltepratze (82a) radial unter der Wirkung des Schubs des Schlagbolzens (60) von einer Rückhalteposition, in der die erste Rampe (72a) in axialer Auflage auf der zweiten Rampe (86a) ist, so dass sich die Rückhaltepratze (82a) dem Schieben des Schlagbolzens (60) widersetzt, bis zu einer Freigabeposition, in der die Rückhaltepratze (82a) abgespreizt wird, um den Schlagbolzen (60) freizugeben, anzutreiben, und
- ein Hindernis (92a), das mit dem Körper (12) fest verbunden und das ausgelegt ist, um die Rückhaltepratze (82a) in ihre Rückhalteposition zu zwingen, wenn der Körper (12) seine Ruheposition belegt, und um die Rückhaltepratze (82a) in ihrer Freigabeposition freizugeben, wenn der Körper (12) seine Einspritzposition belegt.

2. Einspritzvorrichtung (10) ohne Nadel nach Anspruch 1, **dadurch gekennzeichnet, dass** sich die erste Rampe (72a) und die zweite Rampe (86a) jeweils insgesamt radial erstrecken, indem sie eine Neigung nach unten aufweisen, die angepasst ist, um es der zweiten Rampe (86a) der Rückhaltepratze (82a) zu erlauben, auf der ersten Rampe (72a) des Dorns (60) zu gleiten und die Rückhaltepratze (82a) zu ihrer Freigabeposition unter der Wirkung des axialen Schubs des Schlagbolzens (60), der von der dazugehörenden Feder (78) angetrieben wird, abzuspreizen.

3. Einspritzvorrichtung (10) ohne Nadel nach einem der vorstehenden Ansprüche, **dadurch gekennzeichnet, dass** der Dorn (70a) des Schlagbolzens (60) eine erste Gegenrampe (74a) aufweist und die Rückhaltepratze (82a) eine zweite Gegenrampe (88a) aufweist, wobei sich die Gegenrampen jeweils insgesamt radial erstrecken, indem sie eine Neigung nach oben aufweisen, die angepasst ist, um es der zweiten Gegenrampe (88a) der Rückhaltepratze (82a) zu erlauben, auf der ersten Gegenrampe (74a) des Dorns (70a) zu gleiten und die Rückhaltepratze (82a) zu ihrer Freigabeposition anzutreiben, wenn der Schlagbolzen (60) gleitend von seiner Schlagposition bis zu seiner Schlagposition angetrieben wird.

4. Einspritzvorrichtung (10) ohne Nadel nach einem der vorstehenden Ansprüche, **dadurch gekennzeichnet, dass** die Haube (46) eine Sohle (48) umfasst, die einen Haubengrund bildet, der sich senkrecht zu der Gleitachse (A) erstreckt, und dadurch, dass sich die Rückhaltepratze (82a) axial entlang der Gleitachse (A) von einer Basis, die auf der Sohle (48) verbunden ist, bis zu einem Kopf (84a), der die zweite Rampe (86a) abgrenzt, erstreckt, wobei die Rückhaltepratze (82a) radial elastisch verformbar ist, um das Abspreizen der Pratze (82a) von ihrer Rückhalteposition bis zu ihrer Freigabeposition zu erlauben.

5. Einspritzvorrichtung (10) ohne Nadel nach Anspruch 4, **dadurch gekennzeichnet, dass** die vorgespannte Feder (78), die mit dem Schlagbolzen (60) zusammenwirkt, axial entlang der Gleitachse (A) zwischen der Sohle (48) der Haube (46) und einer Auflagefläche (66) des Schlagbolzens (60) eingefügt ist.

6. Einspritzvorrichtung (10) ohne Nadel nach Anspruch 4, **dadurch gekennzeichnet, dass** die Rückhaltepratze (82a) aus Plastikwerkstoff hergestellt ist, der aus einem Stück mit der Sohle (48) der Haube (46) besteht.

7. Einspritzvorrichtung (10) ohne Nadel nach einem der vorstehenden Ansprüche, **dadurch gekennzeichnet, dass** der Körper (12) eine Führungshülse (90) des Schlagbolzens (60) abgrenzt, die eine insgesamt röhrenförmige Form entlang der Gleitachse (A) aufweist und ausgelegt ist, um den Schlagbolzen (60) axial zwischen seiner Ruheposition und seiner Schlagposition zu führen.

8. Einspritzvorrichtung (10) ohne Nadel nach Anspruch 7, **dadurch gekennzeichnet, dass** die Führungshülse (90) mindestens eine axiale Rille (92a) abgrenzt, die das Hindernis (92a) der Rückhaltepratze (82a) bildet und angepasst ist, um eine radiale Auflage eines axialen freien Endes der Rückhaltepratze (82a) zu bilden.

9. Einspritzvorrichtung (10) ohne Nadel nach einem der vorstehenden Ansprüche, **dadurch gekennzeichnet, dass** ein axiales freies Ende des Schlagbolzens (60) einen Zapfen (68) bildet, der angepasst ist, um das Zündelement (18) des Gasgenerators (16) zu schlagen und zu zünden.

10. Einspritzvorrichtung (10) ohne Nadel nach einem der vorstehenden Ansprüche, **dadurch gekennzeichnet, dass** die Schlagvorrichtung (14) eine symmetrische Auslegung entlang einer axialen Ebene (P), die durch die Gleitachse (A) durchgeht, aufweist, wobei die Vorrichtung (14) durch Symmetrie zwei Rückhaltepratzen (82a, 82b) umfasst, die eine Zange bilden, die jeweils mit einem Dorn (70a, 70b), der zu dem Schlagbolzen (60) gehört, zusammenwirken.

11. Einspritzvorrichtung (10) ohne Nadel nach einem der vorstehenden Ansprüche, **dadurch gekennzeichnet, dass** der Wirkstoff (26), der in dem Behälter (24) enthalten ist, aus der Gruppe ausgewählt ist, die die folgenden Wirkstoffe umfasst:
- Methotrexat,
- Adrenalin,
- Sumatriptan,
- Hydrocortison,
- Naloxon,
- Midazolam,
- Apomorphin,
- Ethylnatrexonbromid,
- Phytomenadion,
- Chlorpromazin-Hydrochlorid,
- Zuclopenthixol-Acetat,
- Danaparoid-Natriuim,
- Enoxaparin-Natrium,
- Estradiolcypionat,
- Medroxyprogesteronacetat,
- Medroparincalcium
- Methylprednisolonacetat,
- Heparincalcium,
- Terbulin.

## Claims

1. A needleless injection device (10) including:
- a cover (46),
- an injection system (28) which comprises at least one piston (32, 34), a reservoir (24) containing an active ingredient, an injection nozzle (38) delimiting at least one injection channel (42),
- a body (12) which is enveloped by the cover (46) and which is slidably mounted relative to the cover (46), from bottom to top along a sliding axis (A), between a rest position and an injection position,
- a gas generator (16) comprising a primer (18), and
- a percussion device (14) that includes a striker (60) slidably mounted along the sliding axis (A) between a rest position in which the striker (60) is blocked in translation by a locking means against a pre-stressed spring (78), and a percussion position in which the striker (60) is released by the locking means to strike the primer (18) of the gas generator (16) under the action of said spring (78)
**characterized in that** the locking means of the striker (60) includes at least:
- a lug (70a) which protrudes from a peripheral face (62) of the striker (60) along an axis generally perpendicular to the sliding axis (A) and which has a first bearing ramp (72a),
- a retaining tab (82a) which is secured to the cover (46) and which has a second bearing ramp (86a) arranged facing said first bearing ramp (72a), said ramps (72a, 86a) being designed to cooperate together in order to radially drive the retaining tab (82a) under the effect of the push of the striker (60), from a retaining position in which the first ramp (72a) axially bears on the second ramp (86a) so that the retaining tab (82a) opposes the sliding of the striker (60) to a release position in which the retaining tab (82a) is spaced apart in order to release the striker (60), and
- an obstruction (92a) which is secured to the body (12) and which is designed to constrain the retaining tab (82a) in its retaining position, when the body (12) occupies its rest position, and to release the retaining tab (82a) in its release position when the body (12) occupies its injection position.

2. The needleless injection device (10) according to claim 1 **characterized in that** the first ramp (72a) and the second ramp (86a) each generally radially extend by having a downward inclination which is adapted to enable the second ramp (86a) of the retaining tab (82a) to slip on the first ramp (72a) of the lug (60) and to space the retaining tab (82a) apart towards its release position, under the effect of the axial push of the striker (60) driven by the associated spring (78).

3. The needleless injection device (10) according to any one of the preceding claims, **characterized in that** the lug (70a) of the striker (60) has a first counter-ramp (74a) and the retaining tab (82a) has a second counter-ramp (88a), said counter-ramps each generally radially extend by having an upward inclination which is adapted to enable the second counter-ramp (88a) of the retaining tab (82a) to slip on the first counter ramp (74a) of the lug (70a) and to drive the retaining lug (82a) towards its release position, when the striker (60) is slidably driven from its rest position, to its percussion position.

4. The needleless injection device (10) according to any one of the preceding claims, **characterized in that** the cover (46) comprises a soleplate (48), forming a cover bottom, which extends perpendicular to the sliding axis (A), and **in that** the retaining tab (82a) axially extends along the sliding axis (A) from a base linked on said soleplate (48), to a head (84a) which delimits the second ramp (86a), the retaining tab (82a) being radially elastically deformable to enable the spacing of the tab (82a) from its retaining position to its release position.

5. The needleless injection device (10) according to claim 4, **characterized in that** the pre-stressed spring (78) which cooperates with the striker (60) is axially interposed along the sliding axis (A), between the soleplate (48) of the cover (46) and a bearing face (66) of the striker (60).

6. The needleless injection device (10) according to claim 4, **characterized in that** the retaining tab (82a) is made of plastic material integral with the soleplate (48) of the cover (46).

7. The needleless injection device (10) according to any one of the preceding claims, **characterized in that** the body (12) delimits a sheath (90) for guiding the striker (60), which has a generally tubular shape along the sliding axis (A) and which is designed to axially guide the striker (60) between its rest position and its percussion position.

8. The needleless injection device (10) according to claim 7, **characterized in that** the guide sheath (90) delimits at least one axial groove (92a) which forms the obstruction (92a) of the retaining tab (82a) and which is adapted to form a radial bearing of an axial free end of the retaining tab (82a).

9. The needleless injection device (10) according to any one of the preceding claims, **characterized in that** an axial free end of the striker (60) forms a stud (68) adapted to strike and initiate the primer (18) of the gas generator (16).

10. The needleless injection device (10) according to any one of the preceding claims, **characterized in that** the percussion device (14) has a symmetrical design along an axial plane (P) passing through the sliding axis (A), said device (14) including, by symmetry, two retaining tabs (82a, 82b) forming a clamp each cooperating with an associated lug (70a, 70b) of the striker (60).

11. The needleless injection device (10) according to any one of the preceding claims, **characterized in that** the active ingredient (26) contained in the reservoir (24) is selected from the group comprising the following active ingredients:
- Methotrexate,
- Adrenaline,
- Sumatriptan,
- Hydrocortisone,
- Naloxone,
- Midazolam,
- Apomorphine,
- Ethylnatrexone bromide,
- Phytomenadione,
- Chlorpromazine hydrocloride,
- Zuclopenthixol acetate,
- Danaparoid sodium,
- Enoxaparin sodium,
- Estradiol cypionate,
- Medoxyprogesterone acetate,
- Medroparin calcium,
- Methylprednisolone acetate
- Heparin calcium,
- Terbulin.
